# EUROPEAN PATENT SPECIFICATION

(11) **EP 3 005 941 B1**
(45) Date of publication and mention of the grant of the patent: **27.12.2017**
(21) Application number: 15187369.2
(22) Date of filing: 30.07.2009
(51) Int. Cl.: A61B 5/05, A61B 5/00, G01N 21/65

(54) **INTRA-OPERATIVE FLUORESCENCE SPECTROSCOPY AND APPLICATIONS OF SAME**
INTRAOPERATIVE FLUORESZENZ-SPEKTROSKOPIE UND ANWENDUNGEN DAVON
SPECTROSCOPIE DE FLUORESCENCE PEROPÉRATOIRE ET SES APPLICATIONS

(30) Priority: 30.07.2008 US 137584 P; 30.07.2008 US 137520 P
(43) Date of publication of application: 13.04.2016
(62) Divisional of application: 09803612.2
(73) Proprietor: Vanderbilt University, Nashville, TN 37212 (US)
(72) Inventor: MAHADEVAN-JANSEN, Anita, Nashville, TN 37235 (US); WHITE, Lisa, Nashville, TN 37212 (US); PHAY, John, Nashville, TN 37212 (US); PARAS, Constantine, A., Nashville, TN 37212 (US); KANTER BARTZ, Elizabeth, Southlake, TX 76092 (US); KELLER, Matthew, D., Nashville, TN 37235 (US); GASPARINO, Nicole, Nashville, TN 37212 (US); WHISENANT, Jennifer, Gray, Nashville, TN 37211 (US); PENCE, Isaac J., Crestwood, 40014 (US)
(74) Representative: Jordan, Volker Otto Wilhelm

(56) References cited:
- US-A1- 2002 177 778
- KOPPANG ET AL: "Canine ceroid-lipofuscinosis - A model for human neuronal ceroid-lipofuscinosis and aging", MECHANISMS OF AGEING AND DEVELOPMENT, ELSEVIER SEQUOIA, LAUSANNE, CH, vol. 2, 1 January 1973 (1973-01-01), pages 421-445, XP023429185, ISSN: 0047-6374, DOI: 10.1016/0047-6374(73)90034-1 [retrieved on 1973-01-01]
- PROSST R L ET AL: "Fluorescence-guided minimally invasive parathyroidectomy: a novel detection technique for parathyroid glands", SURGICAL ENDOSCOPY ; AND OTHER INTERVENTIONAL TECHNIQUES OFFICIAL JOURNAL OF THE SOCIETY OF AMERICAN GASTROINTESTINAL AND ENDOSCOPIC SURGEONS (SAGES) AND EUROPEAN ASSOCIATION FOR ENDOSCOPIC SURGERY (EAES), SPRINGER-VERLAG, NE, vol. 20, no. 9, 26 May 2006 (2006-05-26), pages 1488-1492, XP019443646, ISSN: 1432-2218, DOI: 10.1007/S00464-005-0471-4

## Description

Some references, if any, which may include patents, patent applications and various publications, are cited in a reference list and discussed in the description of this invention. The citation and/or discussion of such references is provided merely to clarify the description of the present invention and is not an admission that any such reference is "prior art" to the invention described herein. In terms of notation, hereinafter, superscript "n" represents the nth reference cited in the reference list. For example, []<6>represents the 6th reference cited in the reference list, namely, Frilling, A. & Weber, F. Complications in Thyroid and Parathyroid Surgery. in Surgery of the Thyroid and Parathyroid Glands 217-224 (2007).

### FIELD OF THE INVENTION

The present invention relates generally to fluorescence spectroscopy, and more particularly to, intra-operative fluorescence spectroscopy and applications of same.

### BACKGROUND OF THE INVENTION

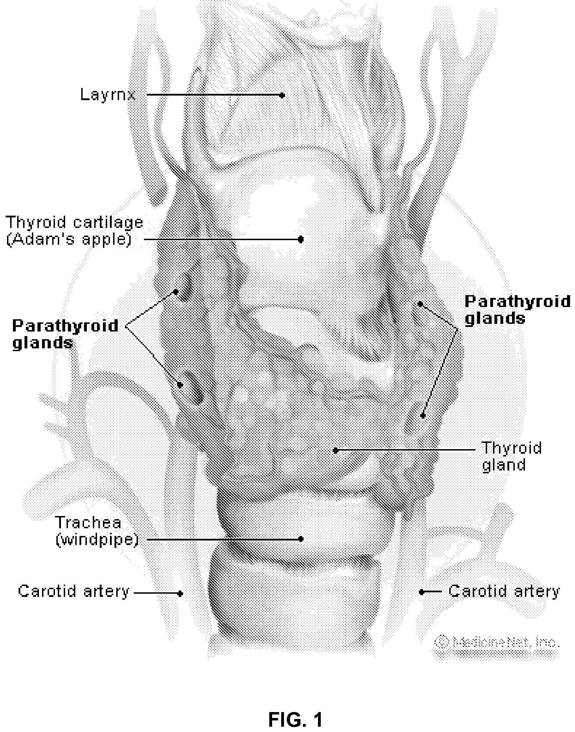

The endocrine system is a complex system of organs and glands which includes the thyroid and parathyroid. The anatomy of the neck is illustrated in Figure 1. The thyroid gland regulates many developmental and metabolic processes. Common diseases of the thyroid include goiters, hyperthyroidism, hypothyroidism, benign and malignant nodules, and autoimmune diseases such as Graves' disease. Surgery is the most common treatment for Graves's disease, goiters, benign thyroid nodules, and thyroid cancers.

The parathyroid lies within the same region as the thyroid in the neck and functions to control calcium levels in the blood. The most common parathyroid disorder is primary hyperparathyroidism, in which one or more of the parathyroid glands become enlarged and hyperactive. This causes excess secretion of parathyroid hormone and a disruption in normal bone and mineral metabolism. The prevalence of primary hyperparathyroidism has been estimated at 21 cases per 100,000 person-years. In 80% of cases, primary hyperparathyroidism is caused by a single overactive parathyroid gland and surgical removal of the diseased parathyroid gland is the only definitive treatment.

Typically there are four tan parathyroid glands, each approximately 6 to 8 mm in size. They are typically positioned within the neck but can vary in location within the body and are sometimes intrathymic. Due to their small size and variability in position, the parathyroid glands are often difficult to distinguish from surrounding tissue and thyroid in the neck. The parathyroid visually resembles its surrounding tissue and this can extend surgical time during a parathyroidectomy, during which the surgeon is simply searching for the small organ. Accidental removal or damage to healthy parathyroids during parathyroid or thyroid surgery can result in serious complications such as hypocalcemia or hypoparathyroidism. Hypoparathyroidism may result from direct injury, devascularization, and/or disruption of the parathyroid glands. Statistics suggest that temporary and permanent hypoparathyroidism rates are between 4-20% during thyroid surgery. The most common complications of both parathyroid and thyroid surgery are postoperative hypocalcemia, vocal-cord paralysis, and hematoma.

The current surgical procedure for thyroid and parathyroid surgeries involves a systematic search within the neck in which the surgeon is mainly relying on visual inspection to identify target tissues. The incidence of complications occurring due to this subjective method is directly proportional to the extent of thyroidectomy and inversely proportional to the experience of the surgeon. The disadvantages to the current method include the lengthy duration of the surgery, the exploratory nature of the surgery, and the lack of preoperative and intra-operative imaging. Confirmation of removal of the diseased parathyroid relies on histopathology or post-operative diagnosis of symptoms.

Moreover, while surgical guidance system has been developed and utilized for brain surgery and other organ surgery procedures, none is available for thyroid and parathyroid surgeries. Therefore, a heretofore unaddressed need exists in the art to address the aforementioned deficiencies and inadequacies.

US 2002177778 A1 discloses a system for detecting amyloidosis in a mammal. The system includes a radiation emitting and collecting system, a light processing unit, a computing device, and a video monitor. Further US 200217778A1 discloses a method of detecting amyloid-containing deposits in a mammal without administering exogenous chemicals such as fluorophores or chromophores. The method includes illuminating a tissue with a light source having a wavelength about 700nm to about 760 nm, and detecting the emitted light of the tissue through a light collection unit. The light collection unit is operatively connected to a light processing unit, wherein the light processing unit is capable of comparing a spectrum of fluorescence from examined tissue to that of known tissue.

KOPPANG ET AL: "Canine ceroid-lipofuscinosis - a model for human neuronal ceroid-lipofuscinosis and aging", MECHANISMS OF AGEING AND DEVELOPMENT, ELSEVIER SEQUIA; LAUSANNE, CH, vol. 2, 1 January 1973, pages 421 - 445, XP023429185, ISSN: 0047-634, DOI: 10.1016/0047-6374(73)90034-1 discloses a method for observing autofluorescent pigment through a fluorescence microscope.

PROSST R L ET AL: "Fluorescence-guided minimally invasive parathyroidectomy: a novel detection technique for parathyroid glands", SURGICAL ENDOSCOPY; AND OTHER INTERVENTIONAL TECHNIQUES OFFICIAL JOURNAL OF THE SOCIETY OF AMERICAN GASTROINTESTINAL AND ENDOSCOPIC SURGEIN (SAGES) AND EUROPEAND ASSOCIATION FOR ENDOSCOPIC SURGERY (EAES), SPRINGER-VERLAG, NE, vol. 20, no. 9, 26 may 2006, pages 1488 - 1492, XP019443646, ISSN: 1432 - 2218, DOI: 10.1007/S00464-005-0471-4 discloses a method and system for detecting parathyroid glands with minimally invasive videoscopically assisted parathyroidectomy after photosensitization with aminolevulinic acid. Further PROSST R L ET AL discloses a method for detecting the photosensitization parathyroid glands by exciting the parathyroid glands with blue light and optaining the emitted light with a CCD camera.

In view of the foregoing it is an object of the present invention to provide a more reliable imaging system to help identify the parathyroid glands intra-operatively.

This object is solved according to the invention by a process as defined in claim 1 and as defined in claim 3. Preferred embodiments of the invention are described in the dependent claims.

### SUMMARY OF THE INVENTION

The present invention allows to provide anatomical guidance in endocrine surgery.

In one embodiment, the illuminating step comprises the step of illuminating human tissues with a beam of light from a laser light source.

In one embodiment, the auto-fluorescent data comprise near-infrared auto-fluorescent data, which can be normalized to produce a normalized intensity spectrum, wherein the peak in the normalized intensity spectrum is produced by the thyroid or parathyroid tissues. Furthermore, the tissues that produce the highest peak in a normalized intensity spectrum corresponding to as the parathyroid tissues, and the tissues that produce the second highest peak in a normalized intensity spectrum corresponding to the thyroid tissues.

A typical system for intra-operatively providing anatomical guidance in endocrine surgery includes a light source for illuminating human tissues in the neck area of a human being in operation with a beam of light, means for obtaining auto-fluorescent data from the light scattered from the human tissues, and a display in communication with the means for obtaining auto-fluorescent data and for displaying images generated from the obtained auto-fluorescent data in a display to provide anatomical guidance in an endocrine surgery.

In one embodiment, the light source comprises a laser light source.

These and other aspects of the present invention will become apparent from the following description of the preferred embodiment taken in conjunction with the following drawings, although variations and modifications therein may be affected without departing from the scope of the novel concepts of the disclosure.

### BRIEF DESCRIPTION OF THE DRAWINGS

Figure 1 shows a schematic of thyroid/parathyroid glands.
Figure 2 schematically shows an excitation system.
Figure 3 shows a layout of NBS 1963 A test card.
Figure 4 is a photo of a system reduced to practice, which is corresponding to a system according to one embodiment of the present invention.
Figure 4A schematically shows a system according to another embodiment of the present invention.
Figure 4B is a photo of a system reduced to practice, which is corresponding to a system according to the embodiment of the present invention as shown in 4A.
Figure 4C schematically shows a system according to yet another embodiment of the present invention.
Figure 4D is a photo of a system reduced to practice, which is corresponding to a system according to the embodiment of the present invention as shown in 4C.
Figure 4E schematically shows a system according to a further embodiment of the present invention
Figure 4F is a photo of a system reduced to practice, which is corresponding to a system according to the embodiment of the present invention as shown in 4E.
Figure 4G schematically shows a system according to yet a further embodiment of the present invention.
Figure 5 shows an image of the glass slide resolution target captured with the CCD camera.
Figure 6 shows an image of tissue taken with the viewer/camera coupled system.
Figure 7 shows a curve of laser power versus its distance from the tissue.
Figure 8 shows images of parathyroid tissue under visible light (left) and fluorescing in the NIR range (right), respectively.
Figure 9 shows normalized signal from parathyroid tissue (dashed line), two thyroid measurements (dotted line) and fat muscle and trachea, respectively, all of which obtained through a method according to one embodiment of the present invention. The parathyroid signal if significantly higher than the anything else in the neck. It is seven times greater than the thyroid and its peak intensity.
Figure 10 shows normalized average parathyroid and thyroid peak intensity across 15 patients, respectively, all of which obtained through a method according to one embodiment of the present invention. In each, the signal arising from the parathyroid measurements is greater than the thyroid ranging from twice as great to more than ten times as strong.
Figure 11 shows *in vitro* fluorescence of parathyroid and thyroid samples obtained from pathology, respectively, all of which obtained through a method according to one embodiment of the present invention. The parathyroid located on the right exhibits much higher overall fluorescence.

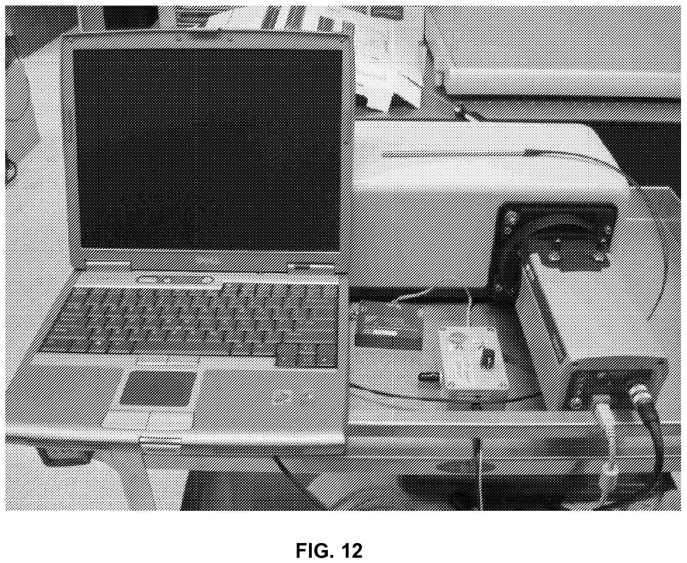

Figure 12 is a photo of a clinical Raman system reduced to practice, which is corresponding to a system according to one embodiment of the present invention.

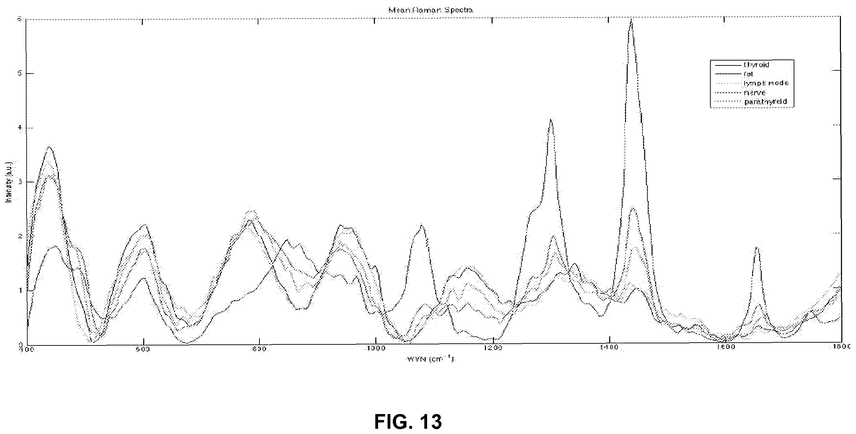

Figure 13 shows mean Raman spectroscopy spectra of various tissue types processed.

### DETAILED DESCRIPTION OF THE INVENTION

The present invention is more particularly described in the following examples that are intended as illustrative only since numerous modifications and variations therein will be apparent to those skilled in the art. Various embodiments of the invention are now described in detail. Referring to the drawings, like numbers indicate like components throughout the views. As used in the description herein and throughout the claims that follow, the meaning of "a", "an", and "the" includes plural reference unless the context clearly dictates otherwise. Also, as used in the description herein and throughout the claims that follow, the meaning of "in" includes "in" and "on" unless the context clearly dictates otherwise. Moreover, titles or subtitles may be used in the specification for the convenience of a reader, which shall have no influence on the scope of the present invention. Additionally, some terms used in this specification are more specifically defined below.

### DEFINITIONS

The terms used in this specification generally have their ordinary meanings in the art, within the context of the invention, and in the specific context where each term is used. Certain terms that are used to describe the invention are discussed below, or elsewhere in the specification, to provide additional guidance to the practitioner regarding the description of the invention. For convenience, certain terms may be highlighted, for example using italics and/or quotation marks. The use of highlighting has no influence on the scope and meaning of a term; the scope and meaning of a term is the same, in the same context, whether or not it is highlighted. It will be appreciated that same thing can be said in more than one way. Consequently, alternative language and synonyms may be used for any one or more of the terms discussed herein, nor is any special significance to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and in no way limits the scope and meaning of the invention or of any exemplified term. Likewise, the invention is not limited to various embodiments given in this specification.

Unless otherwise defined, all technical and scientific terms used herein have the same meaning as commonly understood by one of ordinary skill in the art to which this invention pertains. In the case of conflict, the present document, including definitions will control.

As used herein, "around", "about" or "approximately" shall generally mean within 20 percent, preferably within 10 percent, and more preferably within 5 percent of a given value or range. Numerical quantities given herein are approximate, meaning that the term "around", "about" or "approximately" can be inferred if not expressly stated.

As used herein, the term "living subject" refers to a human being such as a patient, or an animal such as a lab testing monkey.

As used herein, the term "Raman spectroscopy" refers to an optical technique that probes the specific molecular content of a sample by collecting in-elastically scattered light. As photons propagate through a medium, they undergo both absorptive and scattering events. In absorption, the energy of the photons is completely transferred to the material, allowing either heat transfer (internal conversion) or re-emission phenomena such as fluorescence and phosphorescence to occur. Scattering, however, is normally an in-elastic process, in which the incident photons retain their energy. In Raman scattering, the photons either donate or acquire energy from the medium, on a molecular level. In contrast to fluorescence, where the energy transfers are on the order of the electronic bandgaps, the energy transfers associated with Raman scattering are on the order of the vibrational modes of the molecule. These vibrational modes are molecularly specific, giving every molecule a unique Raman spectral signature.

Raman scattering is a very weak phenomena, and therefore practical measurement of Raman spectra of a medium requires high power excitation laser sources and extremely sensitive detection hardware. Even with these components, the Raman spectra from tissue are masked by the relatively intense tissue auto-fluorescence. After detection, post processing techniques are required to subtract the fluorescent background and enable accurate visualization of the Raman spectra. Raman spectra are plotted as a function of frequency shift in units of wavenumber (cm⁻¹). The region of the Raman spectra where most biological molecules have Raman peaks is from 500 to 2000 cm⁻¹. In contrast to fluorescence spectra, Raman spectra have sharp spectral features that enable easier identification of the constituent sources of spectral peaks in a complex sample. In the context of detecting the changes that cancerous tissues undergo, differences in the Raman spectral features that correlate to the increased nucleic acid content in neoplastic cells has observed.

The term "fluorescence spectroscopy" or its acronym "fluorometry" or "spectrofluorometry" refers to a type of electromagnetic spectroscopy which analyzes fluorescence from a sample. It involves using a beam of light, usually ultraviolet light, that excites the electrons in molecules of certain compounds and causes them to emit light of a lower energy, typically, but not necessarily, visible light. A complementary technique is absorption spectroscopy.

Fluorescence spectroscopy utilizes that fact that molecules have various states referred to as energy levels. Fluorescence spectroscopy is primarily concerned with electronic and vibrational states of energy. Generally, the sample being examined will have a ground electronic state (a low energy state) of interest, and an excited electronic state of higher energy. Within each of these electronic states are various vibrational states.

In fluorescence spectroscopy, the sample is first excited, by absorbing a photon, from its ground electronic state to one of the various vibrational states in the excited electronic state. Collisions with other molecules cause the excited molecule to lose vibrational energy until it reaches the lowest vibrational state of the excited electronic state.

The molecule then drops down to one of the various vibrational levels of the ground electronic state again, emitting a photon in the process. As molecules may drop down into any of several vibrational levels in the ground state, the emitted photons will have different energies, and thus frequencies. Therefore, by analysing the different frequencies of light emitted in fluorescent spectroscopy, along with their relative intensities, the structure of the different vibrational levels can be determined.

In a typical measurement, the different frequencies of fluorescent light emitted by a sample are measured, holding the excitation light at a constant wavelength. This is called an emission spectrum. An excitation spectrum is measured by recording a number of emission spectra using different wavelengths of excitation light.

As used herein, the term "auto-fluorescence" refers to the fluorescence of other substances than the fluorophore of interest. It increases the background signal. Auto-fluorescence can be problematic in fluorescence microscopy. In most fluorescence microscopy, fluorescent stains (such as fluorescently-labeled antibodies) are applied to samples to stain specific structures. Auto-fluorescence interferes with detection of the resulting specific fluorescent signals, especially when the signals of interest are very dim - it causes structures other than those of interest to become visible. Depending upon the shape of the structures of interest and the other structures, it may not be obvious that this has occurred. In some microscopes (mainly confocal microscopes), it is possible to make use of different lifetime of the excited states of the added fluorescent markers and the endogenous molecules to exclude most of the auto-fluorescence.

In a few cases, auto-fluorescence may actually illuminate the structures of interest, or serve as a useful diagnostic indicator such as in the case of "biological auto-fluorescence", which refers to the fact that cells contain molecules, which become fluorescent when excited by UV/V is radiation of suitable wavelength. This fluorescence emission, arising from endogenous fluorophores, is an intrinsic property of cells and is called auto-fluorescence to be distinguished from fluorescent signals obtained by adding exogenous markers. The majority of cell auto-fluorescence originates from mitochondria and lysosomes. Together with aromatic amino acids and lipo-pigments, the most important endogenous fluorophores are pyridinic (NADPH) and flavin coenzymes. In tissues, the extracellular matrix often contributes to the auto-fluorescence emission more than the cellular component, because collagen and elastin have, among the endogenous fluorophores, a relatively high quantum yield. Changes occurring in the cell and tissue state during physiological and/or pathological processes result in modifications of the amount and distribution of endogenous fluorophores and chemical-physical properties of their microenvironment. Therefore, analytical techniques based on auto-fluorescence monitoring may be utilized in order to obtain information about morphological and physiological state of cells and tissues. Moreover, auto-fluorescence analysis can be performed in real time because it does not require any treatment of fixing or staining of the specimens. In the past few years spectroscopic and imaging techniques have been developed for many different applications both in basic research and diagnostics. [*See* Biotechnol Annu Rev. 2005;11:227-56. Cell and tissue autofluorescence research and diagnostic applications. Monici M.] The present invention provides a case where auto-fluorescence finds good use.

As used herein, "charge-coupled device" or "CCD" refers to an analog shift register that enables the transportation of analog signals (electric charges) through successive stages (capacitors), controlled by a clock signal. Charge-coupled devices can be used as a form of memory or for delaying samples of analog signals. Today, they are most widely used in arrays of photoelectric light sensors to serialize parallel analog signals. In a CCD for capturing images, there is a photoactive region (an epitaxial layer of silicon), and a transmission region made out of a shift register (the CCD, properly speaking).

An image is projected through a lens onto the capacitor array (the photoactive region), causing each capacitor to accumulate an electric charge proportional to the light intensity at that location. A one-dimensional array, used in line-scan cameras, captures a single slice of the image, while a two-dimensional array, used in video and still cameras, captures a two-dimensional picture corresponding to the scene projected onto the focal plane of the sensor. Once the array has been exposed to the image, a control circuit causes each capacitor to transfer its contents to its neighbor (operating as a shift register). The last capacitor in the array dumps its charge into a charge amplifier, which converts the charge into a voltage. By repeating this process, the controlling circuit converts the entire semiconductor contents of the array to a sequence of voltages, which it samples, digitizes and stores in some form of memory.

As used herein, "plurality" means two or more.

As used herein, the terms "comprising," "including," "carrying," "having," "containing," "involving," and the like are to be understood to be open-ended, i.e., to mean including but not limited to.

### OVERVIEW OF THE INVENTION

As set forth above, thyroid and parathyroid diseases combine the fields of endocrinology and oncology leading to a complex combination of conditions ¹. When the disease cannot be treated by other methods, surgical means are used to remove the diseased gland(s). The thyroid gland, parathyroid glands, nerves, adipose tissue, and lymph nodes are closely positioned in the neck region. Due to their close proximity and tendency to blend into each other, many of these structures, specifically the parathyroid glands, are difficult to distinguish visually during endocrine surgery. The situation is further complicated by its small size and variability in position. Surgeons must ultimately rely on visual inspection to identify the different tissues, which can be subjective and often inconclusive^{2,3}. Complications occur when the parathyroid is accidentally injured or removed during thyroidectomies or only partially removed in the case of parathyroidectomies⁴.

Existing methods for identifying parathyroid glands are limited and have deficiencies that can result in surgical complications⁵. Current technology relies on histopathology or post-operative diagnosis of symptoms to determine if the parathyroid was accidentally or incompletely removed⁶. An accurate, automated diagnostic method could allow faster, more effective patient management^{7,8}. Optical spectroscopy can detect differences in tissue architecture and biochemical composition. In particular, fluorescence spectroscopy has been of considerable interest in the development of new clinical diagnostic tools. Fluorescence measurements of human tissue can be made in real-time, without tissue removal and diagnosis based on tissue fluorescence can be easily automated⁹. Fluorescent molecules typically exhibit signal in the UV/VIS wavelengths, or about 400 - 700 nm¹⁰. As excitation wavelengths become longer, auto-fluorescence decreases¹⁰. There are no published accounts of near infrared auto-fluorescence being used as a diagnosic tool, and there is currently no real-time method for identifying parathyroid tissue intra-operatively.

It is believed that for the first time, near-infrared wavelengths have been utilized in the present invention and attractive to the inventors due to their increased penetration depth in biological tissues.

In one aspect, the present invention provides, among other things, a process or method of using near-infrared auto-fluorescence for the identification of the parathyroid in vivo during endocrine surgery. Such a method could prevent accidental or incomplete removal of parathyroid glands.

In another aspect, the present invention provides real time imaging systems that can detect the near infrared auto-fluorescence from the tissues in the neck in real time to guide surgical resection or surgical procedure.

In one embodiment, a spectral imaging system, which provides x-y spatial imaging with spectral information in the z direction, was built to detect near-infrared auto-fluorescence. The system includes a near infrared light source for emitting a beam of near-infrared light, a light guide or optical fiber(s) that delivers the light to the tissue, a camera mounted on a stand or to an optical microscope, with a variable filter that can select emission wavelengths, and a computer with a controller in communication with each component/device of the system to coordinate the operation. In operation, tissue was placed under the camera, the near infrared auto-fluorescence imaging signals from the tissue were collected and acquired through the camera, and stored in a memory for further processing.

In another embodiment, an imaging system provides an x-y image (or video image) of the field at a given NIR wavelength with the sensitivity needed for tissue fluorescence measurements. An exemplary device was built with a FIND-R-SCOPE®. Such a system can be made as a portable and hand-held device, in which the image is viewed through a built-in viewer by a user such as a surgeon to see the auto-fluorescence from the tissue or from the targeted area of the tissues on site. The viewer can also be connected to a display with or without a CCD camera attachment. The imaging system can be used to guide the surgeon intra-operatively through a surgical procedure to identify the tissues that are need to be acted on.

In short, according to several embodiments of the present invention, near-infrared (NIR) fluorescence is used to identify parathyroid glands during thyroid and parathyroidectomies. The present invention utilized the discovery that parathyroid tissue had unique optical signals to identify the parathyroid glands and differentiate them from other tissues in the neck intra-operatively, in which the intensity of the parathyroid signal was compared to the fluorescence of the surrounding tissue including the thyroid gland. Several fluorescence measurements were conducted according to the present invention on patients who were undergone endocrine surgery at the Vanderbilt University Medical Center. In each of the measurements, the parathyroid had markedly higher levels of fluorescence. These results indicate that NIR fluorescence provides an excellent tool to locate parathyroid tissue in endocrine surgery.

Further description will be made as to the embodiments of the present invention in conjunction with the accompanying drawings of Figs. 1-11.

### IMPLEMENTATIONS AND EXAMPLES OF THE INVENTION

Without intent to limit the scope of the invention, exemplary methods and their related results according to the embodiments of the present invention are given below. Note that titles or subtitles may be used in the examples for convenience of a reader, which in no way should limit the scope of the invention. Moreover, certain theories are proposed and disclosed herein; however, in no way they, whether they are right or wrong, should limit the scope of the invention so long as the invention is practiced according to the invention without regard for any particular theory or scheme of action.

### EXAMPLE 1

This example in part illustrates a method or process, with corresponding apparatus or system, according to one embodiment of the present invention.

A system according to one embodiment of the present invention allows a surgeon to correctly differentiate between thyroid and parathyroid tissue intra-operatively and immediately. Due to their differing optical properties, thyroid, parathyroid and fat can be easily differentiated intra-operatively by the system. When a 785 nm laser excites a neck area of tissue, the parathyroid fluoresces with a greater intensity than the surrounding thyroid tissue. Adipose tissue will not create detectable fluorescence. Both the thyroid and parathyroid fluoresce at about 822 nm, with the parathyroid fluorescing with greater intensity. The system described in this Example allows the 822 nm light created by the thyroid and parathyroid to be viewed directly by the surgeon as visible light. The parathyroid visualization unit according to one embodiment of the present invention is easy to use, requiring as little training as possible. It also effectively decreases operative time and the number of unsuccessful operations, thereby greatly reducing the need for costly secondary operations. The system may be coupled with equipment already present in the operating room ("OR").

There are currently no methods consistently used intra-operatively to differentiate between thyroid and parathyroid tissue. The system according to one embodiment of the present invention has following features: it uses optical fibers running from a light source such as a 785 nm laser source to excite the tissue, and return fibers running to a detector to detect and quantify the intensity of the fluorescence coming off of the tissue. The system has a display device, allowing for the surgeon to view the differing fluorescence of the two or more tissue types, instead of interpreting a numerical intensity value. The system is attachable to a head-unit that the surgeon could wear during surgery.

The new system is in real-time. This real-time factor could decrease time in the OR by creating an intra-operative imaging modality. This would eliminate the need to send tissue samples to histology during surgery and thus reduce any wait times associated with this. The system is adapted to be able to excite an area equal to the size of the surgeon's incision. It was determined that expanding the excitation beam to 1 cm² would create a beam with adequate power to excite the parathyroid tissue yet was still great enough in size to also excite the surrounding tissue.

***Methodology***. To allow the system according to one embodiment of the present invention has visualization ability, several cameras and viewers were tried and/or evaluated to determine which would be most suitable for this feature. A viewer system with peak sensitivity around 825 nm and the ability to visually show the surgeon the NIR light within the visual spectrum was needed. The FIND-R-SCOPE® Infrared Viewer Model 84499A was the final choice. This viewer has spectral sensitivity from 350 to 1350 nm with peak sensitivity at 800 nm, very close to the 822 nm fluorescence coming from the parathyroid tissue. This model is also lightweight, a feature preferred for attachment to a head-unit. To view only the fluorescence of the tissue, an 820 nm long pass filter was placed in front of the viewer. This was important because it removed the high intensity 785 nm laser light as well as visible light and would reduce any background signal from overhead lights during surgery.

Additionally, the different means of exciting a larger area of tissue than a laser independently would excite tried and/or evaluated. The diameter of the laser beam was measured at approximately 0.3 cm which is rather smaller than the desired one square centimeter. A liquid light guide was utilized because it would excite the desired area of tissue while at the same time, maintain the power necessary to excite the tissue. A liquid light guide from Newport® (Model: 77639 Liquid Light Guide, 420-2000 nm, 0.3 in Core, 3.9 in Bend Radius, 79 in Length) was used in practice. This liquid light guide is compatible with light in the NIR, has a great enough numerical aperture such that a fiber from the laser could be directly coupled into the liquid light guide, and is long enough to allow the laser source to be separate from the excitation-detection system. Other liquid light guides having comparable parameters can also be utilized to practice the present invention.

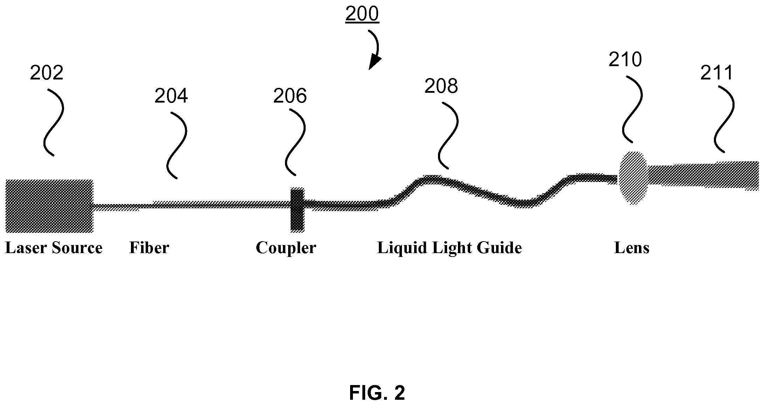

The excitation setup of the system according to one embodiment of the present invention, as shown in Figure 2, used a 785 nm laser coupled to an optical fiber with a diameter of 400 µm and a numerical aperture of 0.22. This cable was then directly coupled to the liquid light guide. The numerical aperture of the liquid light guide is greater than that of the fiber and thus no optics were needed to couple the fiber to the liquid light guide. A Newport® lens with a focal length of 25.4 mm was used at an approximate distance of 6 cm from the liquid light guide to focus the beam. The diameter of the beam when focused was approximately 1.2 cm.

Rather than creating a head unit with a NIR viewer that a surgeon could flip in front of his eyes, a second option was investigated to couple the viewer to a camera and then view the fluorescing tissue on an external screen. A Sony® SC-75 CCD video camera module coupled with the FIND-R-SCOPE® and Pinnacle® software was used to view the fluorescing tissue on an external screen. This setup was also used to take pictures of fluorescence.

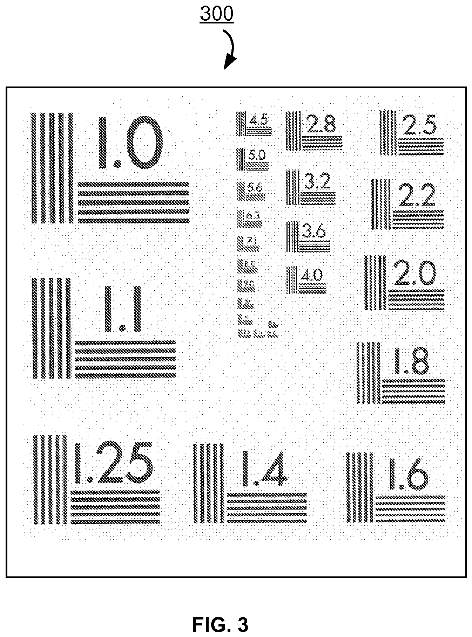

***Spatial Resolution Test.*** In order to quantify the spatial resolution of the viewer, the NBS 1963A spatial resolution test card shown in Figure 3 was used. The test card was placed on a reflectance pad, a light source was used to provide adequate lighting, and the viewer was then focused to determine the spatial resolution of the viewer. The spatial resolution was defined as the smallest set of line pairs per mm that the viewer could differentiate. Additionally, the spatial resolution of the coupled viewer/camera system was tested. Again, the test card was placed on a reflectance pad, a light source was used to provide adequate lighting, and the viewer/camera system attempted to focus the on decreasingly smaller line pairs.

***Spectral Resolution Test.*** Next, the minimum power at which the viewer can detect light at 785 nm was tested. The fiber from the laser source was shown onto the reflectance pad and the viewer was looked through while power was lowered. The decrease in power was stopped when the spot could no longer be seen through the viewer. A power-meter was used to determine the power of the laser at this point. This test yielded the spectral sensitivity of the viewer. The minimum power at which the viewer/camera system can detect 785 nm light was also tested using the same method.

***Power versus Distance Test***. The next test performed was to determine the relationship between power and distance from the liquid light guide. In a darkened room, the power was measured starting with the light source 21.5 cm from the power meter. The power was measured at approximately one-centimeter intervals with the closest measurement made at a distance of 5.5 cm.

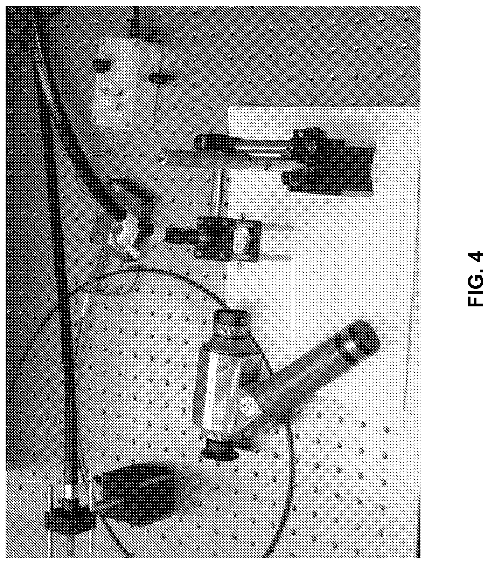

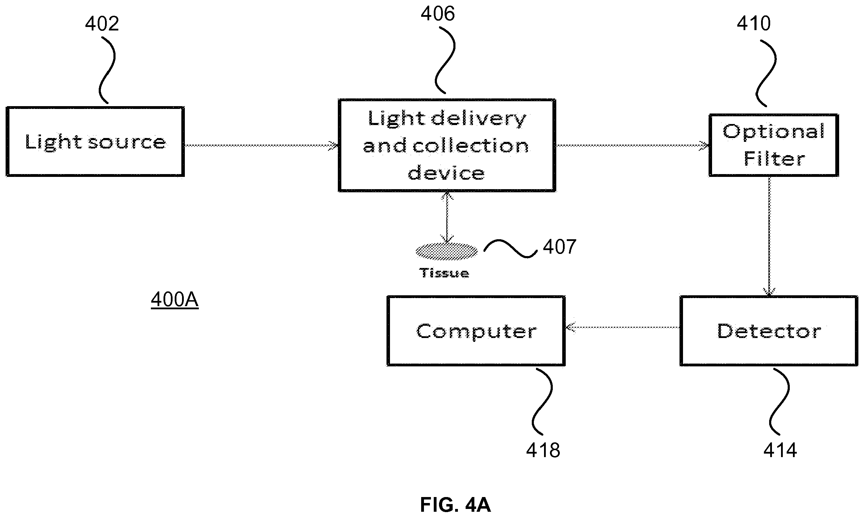

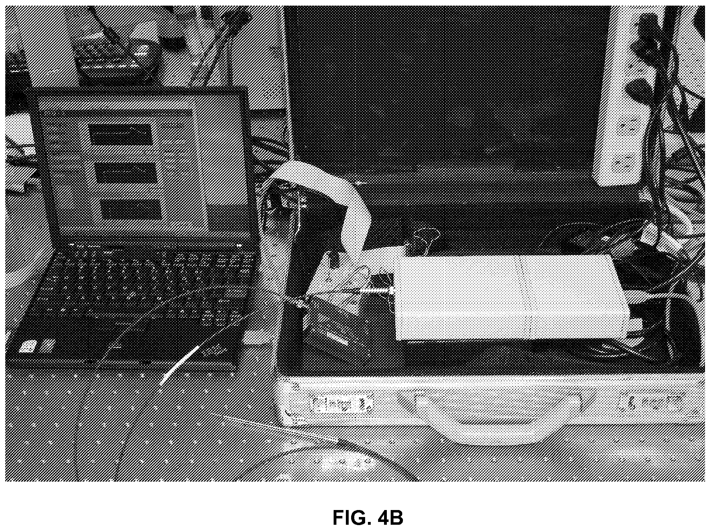

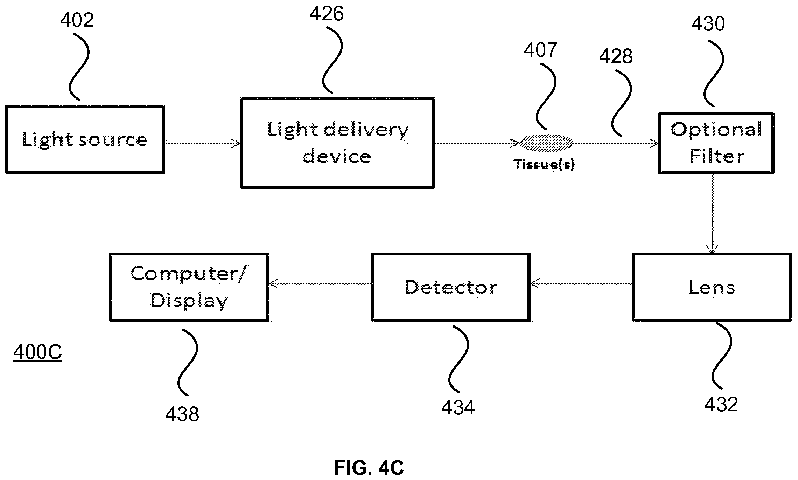

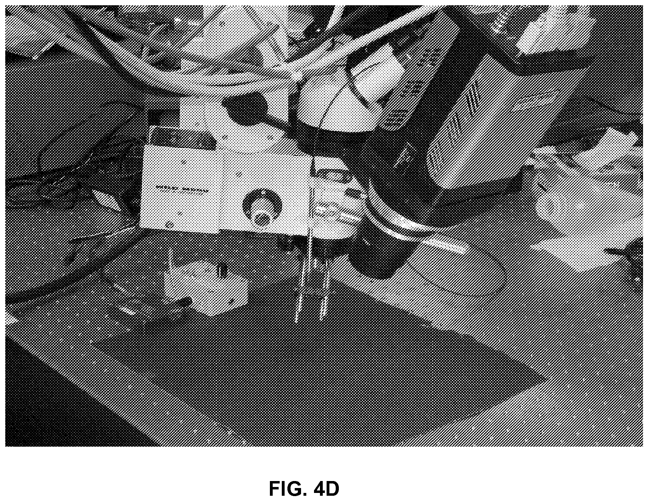

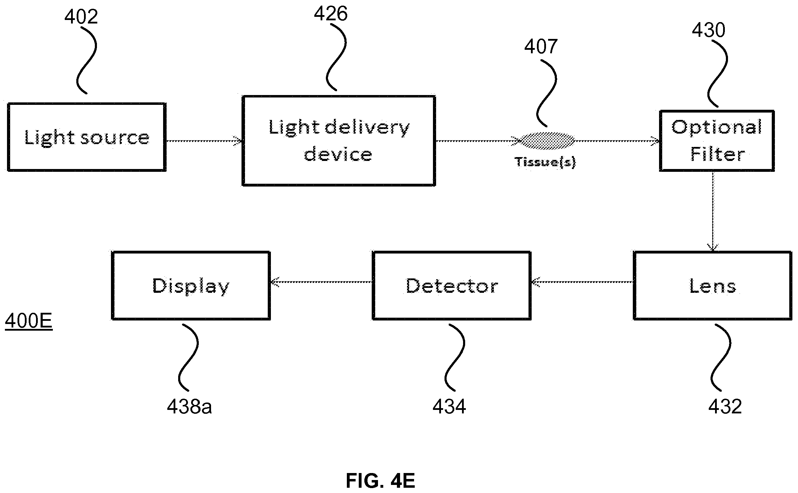

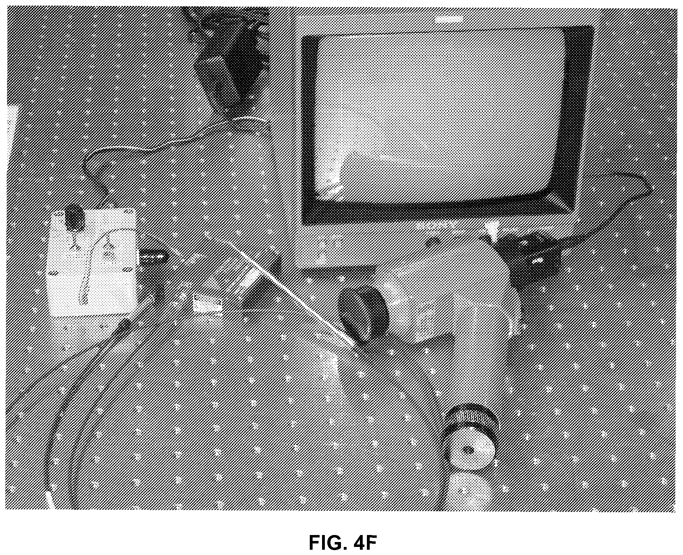

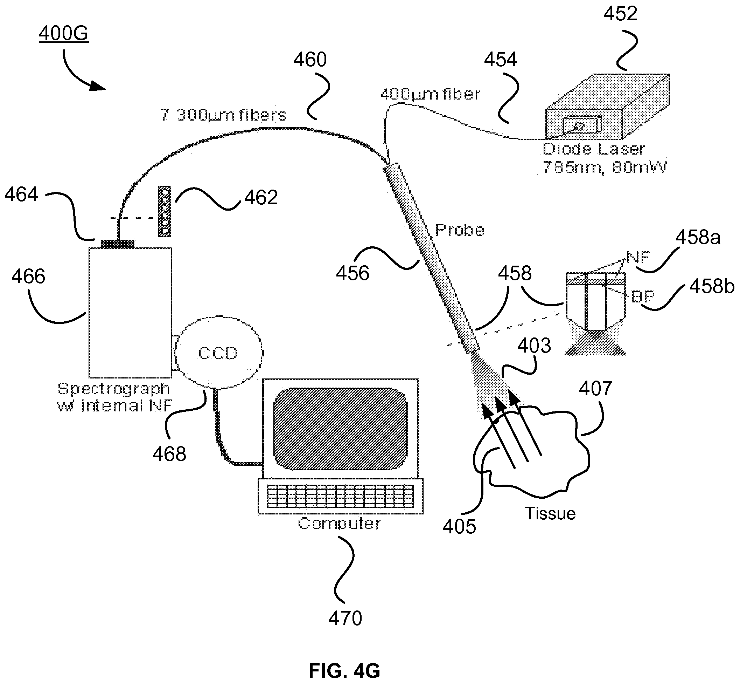

***Tissue Fluorescence Comparison.*** Finally the complete system as photoed in Figure 4 was tested. A moveable arm was built and coupled to the liquid light guide. A cage was built in front of the liquid light guide that created a simple reference to ensure that the light from the liquid light guide was properly focused. This excitation system was used to excite human tissue samples of thyroid and parathyroid. The fluorescence of both the thyroid and the parathyroid were viewed using the viewer and pictures were taken using the camera/viewer-coupled system.

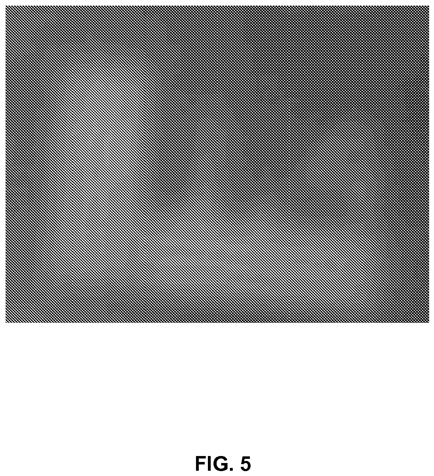

***Spatial Resolution Test Result.*** Looking directly through the FIND-R-SCOPE viewer, we observed a spatial resolution of approximately 3.6 line pairs per millimeter. The inverse of line pairs per millimeter indicates the smallest structure distinguishable by the system. The viewer can distinguish structures as small as 0.28 mm in size. Viewing through the CCD camera connected to an external monitor, the spatial resolution was reduced to approximately 1.4 line pairs per millimeter. The camera/viewer-coupled system can view structures as small as 0.71 mm in size. Figure 5 is an image of the spatial resolution test card using the CCD camera.

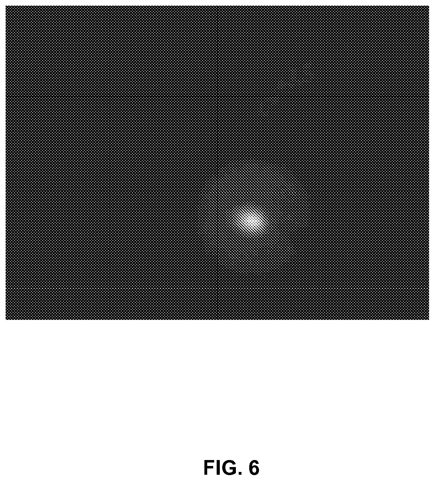

***Spectral Resolution Test Result***. The minimum power at which near-infrared light could be viewed using the viewer was determined to be 3 nW. The spectral resolution of the viewer/camera-coupled system was found to be 8 nW. Figure 6 is a picture taken with the viewer/camera-coupled system.

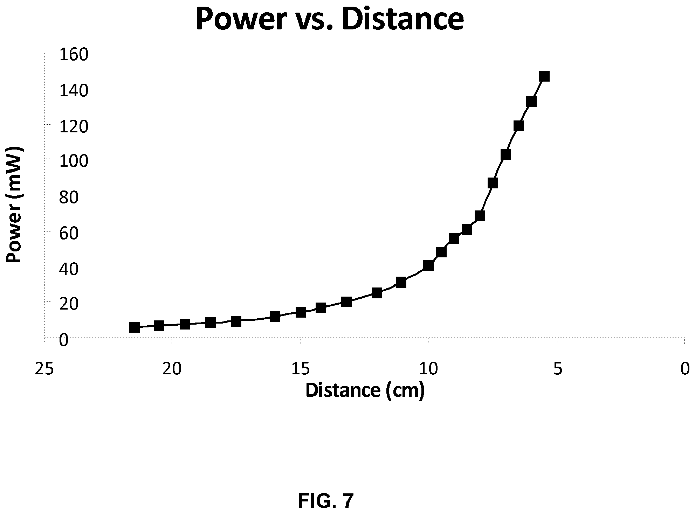

***Power versus Distance Test Result***. The power of the excitation source versus the distance of the laser from the power reader is given in Figure 7. The power needed for tissue excitation is reached at approximately 120 mW at a distance of 6 cm.

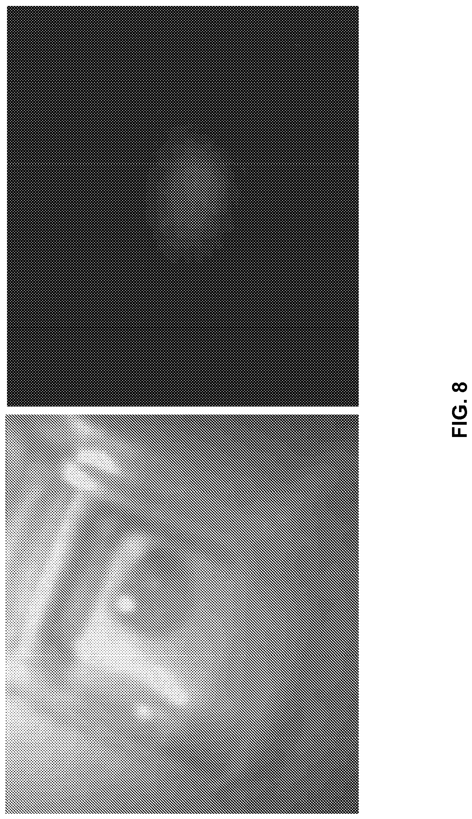

***Tissue Fluorescence Comparison.*** While simultaneously exciting the thyroid and parathyroid, fluorescence was observed coming from the parathyroid but not the thyroid. Figure 8 shows an image of the tissue captured under normal overhead lighting as well as an image of the fluorescing tissue with all wavelengths below 820 nm being filtered out by the 820 nm long pass filter.

***Viewing Fluorescing Tissue.*** The filter applied to the viewer blocked all wavelengths below 820 nm. Because the excitation laser used operates at a 785 nm, any light from the laser was blocked by the filter, and it cannot be seen. However, parathyroid tissue, which emits fluorescence at about 822 nm, was still visible through the device. As stated previously, parathyroid tissue fluoresces at a much greater intensity than fat or thyroid tissue. The human eye cannot differentiate the parathyroid from surrounding tissue because the parathyroid fluoresces at a wavelength outside of the visible spectrum. This system would allow a surgeon to visually differentiate between tissue types based on fluorescence within the NIR range.

***System Limitations and Revolution.*** According to the results of both the spectral and spatial resolution tests, it can be concluded that the resolution of the entire system is mostly limited by the resolution of the CCD camera. In both cases, the resolution observed with the camera attached was significantly lower when viewed with the camera than when viewed directly through the viewer. While the CCD camera can accurately locate fluorescing parathyroid tissue, the system could be improved with a better camera. The system that is not coupled to the CCD camera had better spatial and spectral resolution and ultimately this is the fashion in which the system will be used. Improving the optics of the viewer will increase the spatial resolution of the viewer. The spectral resolution for the viewer alone of 3 nW is very good, and 8 nW spectral resolution of the viewer/camera system is adequate. Overall the system can detect fluorescing parathyroid when using the viewer as well as when using the camera/viewer-coupled system. The current system does not have the sensitivity to view fluorescing thyroid.

***Parathyroid Location In Vivo***. The average human parathyroid measures 5 x 3 x 1 mm⁹. Using only the viewer, the device is capable of achieving a spatial resolution as good as 0.28 mm. Even with the current camera, the observed resolution of approximately 0.71 mm would still be adequate for locating a parathyroid gland that is smaller than average. Hence, the device could provide useful complimentary information to currently employed imaging methods for locating parathyroid tissue when operating in the neck region. The spatial resolution should be improved in the next generation device however because distinguishing the boundaries of the fluorescing parathyroid can be difficult with the current system.

***Physical Specifications of the Device.*** The system's small size and mobility make it adaptable for different operating room layouts. With further work, the device could easily be integrated with existing surgical equipment. The fact that it can be moved easily from place to place makes it cost-effective, as it reduces the need for the purchase of multiple viewing systems to be used by a single hospital.

***Some Practical Applications.*** For use of the lens and laser system in surgery, in order to adequately excite the tissue, the end of the liquid light guide will need to be held within this distance of 6 cm. According to the expert advice, all non-sterile equipment must be held at least 12 inches away from the sterile field. Thus, to maintain a safe, sterile environment, the liquid light guide will require sterilization with each use, and the cord should be covered in sterile material.

Thus, a system according to one embodiment of the present invention as illustrated in Example 1 is proved that by using the fluorescent properties of the thyroid and parathyroid the system is able to visually differentiate the different tissue types during thyroid and parathyroid surgeries. This system can be developed into a full surgical system that can be installed into operating rooms. Improvements including increased spatial and spectral resolution for both the viewer and any secondary camera system, as well as a fully head mountable detector, viewer and excitation system can be made under the principle of the present invention. This system will help to reduce surgical time and the need for secondary surgeries because the system will allow the surgeon to quickly and efficiently differentiate between fat, thyroid and parathyroid. This visual cue will help the surgeon quickly differentiate between tissue types and possibly remove the need for sending tissue samples to histology during surgery. This will reduce time in surgery and will ensure that the correct tissue is removed eliminating the need for secondary surgeries. Other possible improvements could include image processing to create a real time color map image of the fluorescent profile.

### EXAMPLE 2

This example in part illustrates a method or process, with corresponding apparatus or system, according to another embodiment of the present invention.

***Clinical measurements.*** Measurements were performed at the Vanderbilt University Medical Center under approval of the Vanderbilt Institutional Review board. A sterilized fiber probe was used to take six measurements at each tissue site at integration times of 300 ms. Background measurements were performed by recording with the fluorescent lights off before the laser was turned on. The surgeon's confidence level for each tissue type was recorded.

***Spectral measurements.*** Samples were placed on a non-reflective surface and excited with a 6 cm diameter beam. Images were taken at 200 ms acquisition time.

***Instruments.*** Tissue was excited with an Innovative Photonic Solutions 785 nm diode laser. Spectra were recorded with an Ocean Optics S2000-FL fluorescence spectrometer. Spectral images were obtained with a Princeton Instruments Photomax 512.

***Statistical Analyses.*** Data was averaged over 6 measurements. The data was then normalized to the average peak thyroid intensity. Pairs within a patient with a right-tailed t-test were compared and considered values of P ≤ 0.05 significant.

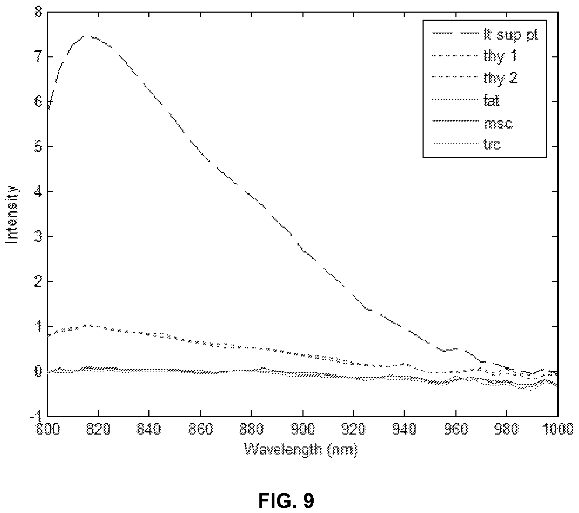

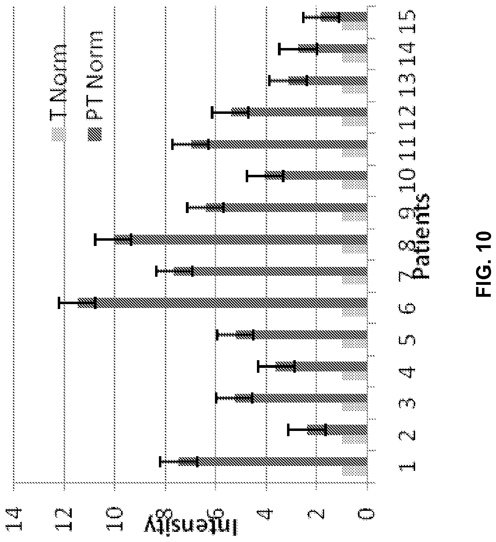

***Fluorescence measurements.*** During the operations, a sterilized optical probe was placed on various tissues in the exposed neck area of a patient and spectral measurements were acquired from each of those sites. The tissue type was noted, as well as the physician's confidence in the investigated sites' histological identity. Spectra were collected using a 300 ms signal collection time. In all cases, the overhead fluorescent lights were turned off during the measurements. Any luminescent lights left on were turned away from the measurement site as they contain spectral components which can interfere with the results. When the tissue was removed in the course of the surgery, specimens from which measurements are taken were then collected for histological identification. All specimens collected were processed and analyzed by a pathologist. Figure 9 depicts a typical spectrum from a patient. The signal from the parathyroid gland has the highest peak intensity and is prominently distinguishable from the other tissues in the neck. The thyroid gland also shows stronger fluorescence than the surrounding muscle and fat. Figure 10 shows the normalized average peak intensity of parathyroid and thyroid measurements from multiple patients. The two glands are compared within each patient. The results are significant with a p-value of 0.000287069. These results clearly illustrate that parathyroid tissue is at least two times more fluorescent than other tissue found in the neck and was as great as eleven times more fluorescent across all patients. Moreover, data was successfully obtained *in vivo*. It is believed that this was the first study performed *in vivo* showing near infrared auto-fluorescence.

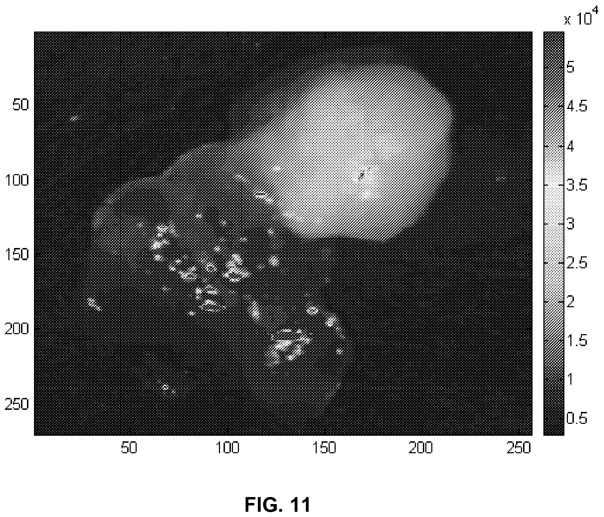

***In vitro* imaging of tissue.** Collecting data in the OR provides *in vivo* fluorescence measurements, however, the tissue is not always easily accessible. This can lead to inconsistent experimental methods between tissue types. To examine the fluorescence of both tissues equally and gauge the potential for visually capturing the intrinsic fluorescence, spectral images were recorded of pathological samples. Samples of thyroid and parathyroid tissue were placed side by side and illuminated with the 785 nm laser. A filter was used to block all reflected light from the laser. Figure 11 shows both glands fluorescing. The parathyroid is on the right and the thyroid on the left. The parathyroid fluoresces just over twice as much as the thyroid tissue with approximate intensities of 23900 and 9999 respectively. Not only was it possible to capture the intrinsic fluorescence with a camera but the parathyroid exhibits stronger fluorescence in *vitro* as well as *in vivo.*

***Understanding the Results***. The results set forth above show that optical spectroscopy according to the present invention can successfully detect parathyroid tissue in endocrine surgery. In each patient the parathyroid signal is greater than the thyroid signal. Moreover, the standard error and p-value show that the parathyroid can be classified with a statistically significant difference each time. This success is very significant for endocrine surgery. Not only can a system according to one embodiment of the present invention discriminate intra-operatively parathyroid glands from the surrounding tissue but it does so with high accuracy. Whereas other intra-operative locaclization methods can be very time consuming and expensive, NIR fluorescence according to the present invention is quick and relatively cheap ^{7,8,11}. This method is better than the current standard of care for multiple reasons. It is fast and safe way of identifying parathyroid glands during surgery. The amount of energy being delivered to the tissue by the laser is low so no damage can occur. Also, it only takes 300 ms to make a measurement and see drastic changes between thyroid and parathyroid fluorescence. The surgeon has a method of identifying parathyroid glands with high accuracy.

NIR fluorescence is not typically seen in biological tissues¹². However, in all patients fluorescence is seen above 800 nm, in the range of about 800 nm to 1,000nm, in the thyroid and parathyroid glands but not in the surrounding tissue. This includes fat, muscle, nerve, trachea and lymph tissue. These results raise several questions. Why is this fluorescence only seen in the parathyroid and thyroid glands? Typically fluorescence arises from conjugated bonds providing an excess of pi electrons¹². Analysis of the tissue and hormone components may be needed to see if the same effect is occurring or if some other phenomenon is responsible. *In vitro* imaging of the tissues confirmed the fluorescence persists even outside of the body. The parathyroid fluoresced twice as strong as the thyroid sample reaffirming that the effect is some inherent property of the tissue. Additionally, there is the potential to take the camera to the OR and create a noncontact method of detecting parathyroid glands intra-operatively, which is further described below according to another embodiment of the present invention.

The largest problem encountered was with the consistency of parathyroid measurements. Because the glands are not always readily accessible it is hard to maintain proper contact between the probe and tissue. In some cases the measurements had to be repeated because the signal weakened over time as indicating that the probe slipped. This introduces a slight bias in that the problem is assumed to be with the probe but the repeat measurements are at a new site and the effect is not seen in easy accessible glands or ex vivo samples. Additionally, different glands within a patient and between patients give variable measurements. Patient to patient variation is expected and corrected by normalizing the data. Differing intensity within the same patient might be explained by accessibility or different amounts of fat and blood over the tissue but because the underlying basis of the signal is not known this is another assumption. With the exception of loss of contact even the lowest parathyroid signal has been higher than the maximum thyroid signal recorded.

The final question is trends in the data. There seems to be no correlation between thyroid or parathyroid signal and age, disease, gender or race. Further patients are required to examine if the signal is random or there is a trend. However, none of these questions challenges the validity and viability of the present invention.

Thus, the results from Example 2 are very promising. NIR fluorescence provides an accurate detection of parathyroid tissue intra-operatively. Additional patient data can be collected to determine the underlying source of fluorescence. A larger patient database may provide more robust results as well as help develop algorithms to detect and identify parathyroid tissue. A larger population of patients may also help determine and trends in the data that would increase the accuracy of algorithms and help elucidate the basis of fluorescence. Additionally, chemical decomposition and in depth analysis of the absorption and fluorescence may help narrow down the reason that only parathyroid and thyroid have strong fluorescence in the neck. Understanding the basis of NIR fluorescence would help identify where the system might fail as well as uncover a range of biological fluorescence that was not even though to really exist. But there is no doubt that Example 2 shows validity and viability of the present invention.

### EXAMPLE 3

This example in part illustrates an apparatus or system, which can be utilized to practice a method or process according to one embodiment of the present invention.

Referring now to Fig. 4A, a system 400A is schematically shown in block diagram. The system 400A has a light source 402, which is designed to provide a beam of light with appropriate excitation wavelengths and energy so as to excite certain molecules of sample tissue 407, when receiving the beam of light, to generate fluorescence emission. The system 400A also has a light delivery and collection device 406, which is optically coupled with the light source 402 and delivers the beam of light from the light source 402 to the tissue 407 and collects optical signals emitted from the tissue 407 responsive to the incident beam of light. An optional filter 410 can be utilized to reduce excess wavelengths and/or ambient light to ensure desirable optical signals to be detected. The system 400A further has a detector 414, which detects and records the optical signals emitted from the tissue. A computer 418 is at least in communication with the detector 414, where the computer 418 is adapted with appropriate controller cards and software to control signal acquisition, process data and display spectra, among other things.

Fig. 4B shows a photo of a system corresponding to system 400A of Fig. 4A, which was reduced to practice.

### EXAMPLE 4

This example in part illustrates an apparatus or system, which can be utilized to practice a method or process according to one embodiment of the present invention.

Referring now to Fig. 4C, a system 400C is schematically shown in block diagram. The system 400C has a light source 402, which is designed to provide a beam of light with appropriate excitation wavelengths and energy so as to excite certain molecules of sample tissue 407, when receiving the beam of light, to generate fluorescence emission. The system 400C also has a light delivery device 426, which is optically coupled with the light source 402 and delivers the beam of light from the light source 402 to the tissue 407. An optional filter 430 can be strategically positioned in an optical path 428 along which optical signals emitted from the tissue 407 responsive to the incident beam of light travel and utilized to reduce excess wavelengths and/or ambient light to ensure desirable optical signals to be detected. The system 400C further has a detector 434, which detects and records the optical signals emitted from the tissue 407. A lens 432 is positioned between the tissue 407 and the detector 434 to receive the optical signals emitted from the tissue and focus them on a desired spot on the detector 434. If an optional filter 430 is utilized, lens 432 will be positioned between the optional filter 430 and the detector 434. A computer 438, which has imaging display capacity or is associated with a display device, is at least in communication with the detector 434, where the computer 438 is adapted with appropriate controller cards and software to control signal acquisition, process data and display spectra, among other things. The display displays instant images processed by the computer 438 from the optical signals emitted from the tissue 407, which may guide a surgeon or a medical professional through a medical procedure to identify tissues and act on them accordingly.

Fig. 4D shows a photo of a system corresponding to system 400C of Fig. 4C, which was reduced to practice.

### EXAMPLE 5

This example in part illustrates an apparatus or system, which can be utilized to practice a method or process according to yet another embodiment of the present invention.

Referring now to Fig. 4E, a system 400E is schematically shown in block diagram. The system 400E has a light source 402, which is designed to provide a beam of light with appropriate excitation wavelengths and energy so as to excite certain molecules of sample tissue 407, when receiving the beam of light, to generate fluorescence emission. The system 400E also has a light delivery device 426, which is optically coupled with the light source 402 and delivers the beam of light from the light source 402 to the tissue 407. An optional filter 430 can be strategically positioned in an optical path 428 along which optical signals emitted from the tissue 407 responsive to the incident beam of light travel and utilized to reduce excess wavelengths and/or ambient light to ensure desirable optical signals to be detected. The system 400C further has a detector 434, which detects and records the optical signals emitted from the tissue 407. A lens 432 is positioned between the tissue 407 and the detector 434 to receive the optical signals emitted from the tissue and focus them on a desired spot on the detector 434. If an optional filter 430 is utilized, lens 432 will be positioned between the optional filter 430 and the detector 434. A display 438a, which has imaging processing and display capacity, is at least in communication with the detector 434, where the display 438a is adapted with appropriate controller cards and software to control signal acquisition, process data and display spectra, among other things. The display 438a displays instant images corresponding to the optical signals emitted from the tissue 407, which may guide a surgeon or a medical professional through a medical procedure to identify tissues and act on them accordingly.

Fig. 4F shows a photo of a system corresponding to system 400E of Fig. 4E, which was reduced to practice.

### EXAMPLE 6

This example in part illustrates an apparatus or system, which can be utilized to practice a method or process according to yet another embodiment of the present invention.

Referring now to Fig. 4G, a system 400G is schematically shown. The system 400G has a light source 452, which is designed to provide a beam of light with appropriate excitation wavelengths and energy so as to excite certain molecules of sample tissue 407, when receiving the beam of light, to generate fluorescence emission. In this embodiment, the light source 42 is a diode laser capable of generating a beam of laser light with wavelength 785nm and power 80mW. Other suitable light sources can also be utilized to practice the present invention.

A first optical fiber 454 optically couples the light source 452 with a probe 456 that functions as a light delivery and collection device. In this embodiment, the first optical fiber 454 is a 400 µm fiber. The probe 456 receives a beam of light 403 from the light source 452 through the first optical fiber 454 and delivers it to the tissue 407 through head portion 458. The head portion 458 also collects optical signals 405 emitted from the tissue 407 responsive to the incident beam of light 403. In one embodiment, the head portion 458 is configured with a noise filter 458a and a band pass 458b to maintain the quality of the optical signals.

A second optical fiber 460 optically couples the probe 456 to means for recording, processing and displaying the optical signals, which, in one embodiment, includes a spectrograph 466, a CCD 468 and a computer 470 with display, where the CCD 468 is in communication with both spectrograph 466 and computer 470. The spectrograph 466 may have internal noise filter installed therein. The second optical fiber 460 is optically coupled with the spectrograph 466 through a coupler 464.

Each of the first optical fiber 454 and the second optical fiber 460 may comprise one or more fibers. In one embodiment, the second optical fiber 460 has 6 or 7 of 300 µm fibers arranged in an array.

CCD 468 and computer 470, adapted with appropriate controller cards and software to control signal acquisition, process data and display spectra, are utilized to process and display images corresponding to the optical signals 405 emitted from the tissue 407, which may guide a surgeon or a medical professional through a medical procedure to identify tissues and act on them accordingly.

System 400G can be adapted as a portable system.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its scope. Accordingly, the scope of the present invention is defined by the appended claims rather than the foregoing description and the exemplary embodiments described therein.

### LIST OF REFERENCES

1. Doherty, G.M. Thyroid and Parathyroid. in Oncology 992-1013 (2006).
2. Bliss, R.D., Gauger, P.G. & Delbridge, L.W. Surgeon's approach to the thyroid gland: surgical anatomy and the importance of technique. World journal of surgery 24, 891-897 (2000).
3. Miller, F.R. Surgical anatomy of the thyroid and parathyroid glands. Otolaryngologic clinics of North America 36, 1-7, vii (2003).
4. ATA. Thyroid Surgery. (American Thyroid Association, 2005).
5. Prosst, R.L., Gahlen, J., Schnuelle, P., Post, S. & Willeke, F. Fluorescence-guided minimally invasive parathyroidectomy: a novel surgical therapy for secondary hyperparathyroidism. Am J Kidney Dis 48, 327-331 (2006).
6. Frilling, A. & Weber, F. Complications in Thyroid and Parathyroid Surgery. in Surgery of the Thyroid and Parathyroid Glands 217-224 (2007).
7. Ahuja, A.T., et al. Imaging for primary hyperparathyroidism--what beginners should know. Clinical radiology 59, 967-976 (2004).
8. Fakhran, S., Branstetter, B.F.t. & Pryma, D.A. Parathyroid imaging. Neuroimaging clinics of North America 18, 537-549, ix (2008).
9. Ramanujam, N., et al. Spectroscopic diagnosis of cervical intraepithelial neoplasia (CIN) in vivo using laser-induced fluorescence spectra at multiple excitation wavelengths. Lasers in surgery and medicine 19, 63-74 (1996).
10. Lakowicz, J.R. Fluorophores. in Principles of Fluorescence Spectroscopy 63-95 (2006).
11. Kim, L.a. A.M. Hyperparathyroidism. (ed. Daniel Einhorn, F.T., Don S Schalch, Mark Cooper and George T Griffing) (Webmd, 2008).
12. Lakowicz, J.R. Introduction to Fluorescence. in Principles of Fluorescence Spectroscopy 1-26 (2006).

## Claims

1. A process for intra-operatively identifying parathyroid tissues of a living subject, comprising the steps of:
(a) illuminating tissues (407) in the neck area of the living subject with a beam of light (403) having a predetermined wavelength in the near-infrared range;
(b) obtaining a near-infrared auto-fluorescence spectrum of intensity from the light emitted from the illuminated tissues (407);
(c) finding the highest peak intensity in the near-infrared auto-fluorescence spectrum; and
**characterized in that** the process further comprises the steps of:
(d) identifying tissues that correspond to the highest peak intensity as parathyroid tissues;
(e) finding the second highest peak intensity in the near-infrared auto-fluorescence spectrum;
(f) identifying tissues that correspond to the second highest peak intensity as thyroid tissues; and
(g) displaying images visually representing the near-infrared auto-fluorescence spectrum in real time to a medical professional.

2. The process of claim 1, wherein the step of obtaining the near-infrared auto-fluorescence spectrum of intensity comprises collecting the light emitted from the illuminated tissues (407) with a collection probe (428, 456) and obtaining the near-infrared auto-fluorescence spectrum with a detector (414, 434, 468).

3. A system (400 A; C; E; G) for intra-operatively identifying parathyroid tissues of a living subject, comprising:
(a) means (402, 452) for illuminating tissues in a target region in the neck area of the living subject;
(b) means (406, 432, 458) for collecting light emitted from the tissues in response to the illumination;
(c) means (414, 434, 466) for obtaining a near-infrared auto-fluorescence spectrum of intensity from the collected light;
(d) means (418, 438, 438a, 470) for finding a highest peak in the near-infrared auto-fluorescence spectrum; and
**characterized in that** the system further comprises:
(e) means (418, 438, 438a, 470) for identifying tissues corresponding to the highest peak as parathyroid tissues;
(f) means (418, 438, 438a, 470) for finding the second highest peak intensity in the near-infrared auto-fluorescence spectrum;
(g) means _(418, 438, 438a, 470) for identifying tissues corresponding to the second highest peak intensity as thyroid tissues; and
(h) means (418, 438, 438a, 470) for displaying images visually representing the near-infrared auto-fluorescence spectrum in real time to a medical professional performing the endocrine surgery.

4. The system (400 A, C; E; G) of claim 3, wherein the means for illuminating the tissues is a light source (402, 452) configured to emit a beam of light (403) having a wavelength in the near-infrared range, further comprising an optical fiber (426, 454) means optically coupled to the light source (402, 452), for receiving the beam of light (403) from the light source (402, 452) and delivering the received beam of light (403) to the tissues (407), and wherein the means for obtaining the near-infrared auto-fluorescence spectrum of intensity comprises a collection probe (428, 456) and a detector (414, 434, 468).

## Patentansprüche

1. Verfahren zum intraoperativen Identifizieren von Nebenschilddrüsengeweben eines lebenden Objekts, umfassend die Schritte:
(a) Beleuchten von Geweben (407) im Halsbereich des lebenden Objekts mit einem Lichtstrahl (403) mit einer festgelegten Wellenlänge im nah-infraroten Bereich;
(b) Erhalten eines nah-infraroten Autofluoreszenz-Spektrums einer Intensität aus dem von den beleuchteten Geweben (407) emittierten Licht;
(c) Finden der höchsten Peak-Intensität in dem nah-infraroten Autofluoreszenz-Spektrum; und
**dadurch gekennzeichnet, dass** das Verfahren ferner die Schritte umfasst:
(d) Identifizieren von Geweben, welche der höchsten Peak-Intensität entsprechen, als Nebenschilddrüsengewebe;
(e) Finden der zweithöchsten Peak-Intensität in dem nah-infraroten Autofluoreszenz-Spektrum;
(f) Identifizieren von Geweben, welche der zweithöchsten Peak-Intensität entsprechen, als Schilddrüsengewebe; und
(g) Anzeigen von Bildern, welche das nah-infrarote Autofluoreszenz-Spektrum visuell repräsentieren, in Echtzeit für einen Mediziner.

2. Verfahren nach Anspruch 1, wobei der Schritt des Erhaltens des nah-infraroten Autofluoreszenz-Spektrums einer Intensität ein Sammeln des von den beleuchteten Geweben (407) emittierten Lichts mit einer Sammelsonde (428, 456) und ein Erhalten des nah-infraroten Autofluoreszenz-Spektrums mit einem Detektor (414, 434, 468) umfasst.

3. System (400 A; C; E; G) zum intraoperativen Identifizieren von Nebenschilddrüsengeweben eines lebenden Objekts, umfassend:
(a) Mittel (402, 452) zum Beleuchten von Geweben in einem Zielbereich im Halsbereich des lebenden Objekts;
(b) Mittel (406, 432, 458) zum Sammeln von von den Geweben als Reaktion auf die Beleuchtung emittiertem Licht;
(c) Mittel (414, 434, 466) zum Erhalten eines nah-infraroten Autofluoreszenz-Spektrums einer Intensität von dem gesammelten Licht;
(d) Mittel (418, 438, 438a, 470) zum Finden eines höchsten Peaks in dem nah-infraroten Autofluoreszenz-Spektrum; und
**dadurch gekennzeichnet, dass** das System ferner umfasst:
(e) Mittel (418, 438, 438a, 470) zum Identifizieren von Geweben, welche dem höchsten Peak entsprechen, als Nebenschilddrüsengewebe;
(f) Mittel (418, 438, 438a, 470) zum Finden der zweithöchsten Peak-Intensität in dem nah-infraroten Autofluoreszenz-Spektrum;
(g) Mittel (418, 438, 438a, 470) zum Identifizieren von Geweben, welche der zweithöchsten Peak-Intensität entsprechen, als Schilddrüsengewebe; und
(h) Mittel (418, 438, 438a, 470) zum Anzeigen von Bildern, welche das nah-infrarote Autofluoreszenz-Spektrum visuell repräsentieren, in Echtzeit für einen Mediziner, welcher die endokrine Operation durchführt.

4. System (400 A; C; E; G) nach Anspruch 3, wobei die Mittel zum Beleuchten der Gewebe eine Lichtquelle (402, 452) sind, welche dazu eingerichtet ist, einen Lichtstahl (403) mit einer Wellenlänge im nah-infraroten Bereich zu emittieren, ferner umfassend ein Lichtleiter (426, 454)-Mittel, welches optisch mit der Lichtquelle (402, 452) gekoppelt ist, zum Aufnehmen des Lichtstrahls (403) von der Lichtquelle (402, 452) und Liefern des aufgenommenen Lichtstrahls (403) zu den Geweben (407), und wobei die Mittel zum Erhalten des nah-infraroten Autofluoreszenz-Spektrums einer Intensität eine Sammelsonde (428, 456) und einen Detektor (414, 434, 468) umfassen.

## Revendications

1. Procédé d'identification peropératoire de tissus parathyroïdiens d'un sujet vivant, comprenant les étapes :
(a) d'éclairage de tissus (407) dans la zone du cou du sujet vivant avec un faisceau de lumière (403) ayant une longueur d'onde prédéterminée dans la plage proche infrarouge ;
(b) d'obtention d'un spectre d'intensité d'auto-fluorescence proche infrarouge de la lumière émise par les tissus éclairés (407) ;
(c) de recherche du pic d'intensité le plus élevé dans le spectre d'auto-fluorescence proche infrarouge ; et
**caractérisé en ce que** le procédé comprend en outre les étapes :
(d) d'identification de tissus qui correspondent au pic d'intensité le plus élevé comme étant des tissus parathyroïdiens ;
(e) de recherche du second pic d'intensité le plus élevé dans le spectre d'auto-fluorescence proche infrarouge ;
(f) d'identification de tissus qui correspondent au second pic d'intensité le plus élevé comme étant des tissus parathyroïdiens ; et
(g) d'affichage d'images représentant visuellement le spectre d'auto-fluorescence proche infrarouge en temps réel à un professionnel de la santé.

2. Procédé selon la revendication 1, dans lequel l'étape d'obtention du spectre d'intensité d'auto-fluorescence proche infrarouge comprend la collecte de la lumière émise par les tissus éclairés (407) avec une sonde de collecte (428, 456) et d'obtention du spectre d'auto-fluorescence proche infrarouge avec un détecteur (414, 434, 468).

3. Système (400 A ; C ; E ; G) d'identification peropératoire de tissus parathyroïdiens d'un sujet vivant, comprenant :
(a) un moyen (402, 452) d'éclairage de tissus dans une région cible dans la zone du cou du sujet vivant ;
(b) un moyen (406, 432, 458) de collecte de la lumière émise par les tissus en réponse à l'éclairage ;
(c) un moyen (414, 434, 466) d'obtention d'un spectre d'intensité d'auto-fluorescence proche infrarouge de la lumière collectée ;
(d) un moyen (418, 438, 438a, 470) de recherche d'un pic le plus élevé dans le spectre d'auto-fluorescence proche infrarouge ; et
**caractérisé en ce que** le système comprend en outre :
(e) un moyen (418, 438, 438a, 470) d'identification de tissus qui correspondent au pic d'intensité le plus élevé comme étant des tissus parathyroïdiens ;
(f) un moyen (418, 438, 438a, 470) de recherche du second pic d'intensité le plus élevé dans le spectre d'auto-fluorescence proche infrarouge ;
(g) un moyen (418, 438, 438a, 470) d'identification de tissus correspondant au second pic d'intensité le plus élevé comme étant des tissus parathyroïdiens ; et
(h) un moyen (418, 438, 438a, 470) d'affichage d'images représentant visuellement le spectre d'auto-fluorescence proche infrarouge en temps réel à un professionnel de la santé réalisant une opération chirurgicale endocrinienne.

4. Système (400 A, C ; E ; G) selon la revendication 3, dans lequel le moyen d'éclairage des tissus est une source de lumière (402, 452) configurée pour émettre un faisceau de lumière (403) ayant une longueur d'onde dans la plage proche infrarouge, comprenant en outre un moyen de fibre optique (426, 454) optiquement couplé à la source de lumière (402, 452), pour recevoir le faisceau de lumière (403) de la source de lumière (402, 452) et délivrer le faisceau de lumière (403) reçu aux tissus (407), et dans lequel le moyen d'obtention du spectre d'intensité d'auto-fluorescence proche infrarouge comprend une sonde de collecte (428, 456) et un détecteur (414, 434, 468).
